# EUROPEAN PATENT APPLICATION

(11) **EP 4 781 924 A1**
(43) Date of publication of application: **29.07.2026**
(21) Application number: 24871881.9
(22) Date of filing: 12.09.2024
(51) Int. Cl.: A61B 17/115

(54) **MEDICAL SHEET AND METHOD FOR PRODUCING MEDICAL SHEET**

(30) Priority: 27.09.2023 JP 2023164236
(71) Applicant: Terumo Kabushiki Kaisha, Tokyo 151-0072 (JP)
(72) Inventor: SEGAMI, Yuuto, Ashigarakami-gun, Kanagawa 259-0151 (JP); KAI, Miho, Ashigarakami-gun, Kanagawa 259-0151 (JP); TAKAHASHI, Akihiro, Ashigarakami-gun, Kanagawa 259-0151 (JP); ARAMAKI, Naoki, Ashigarakami-gun, Kanagawa 259-0151 (JP)
(74) Representative: Casalonga
(86) International application number: PCT/JP2024/032650
(87) International publication number: WO 2025/070084

(57) **Abstract**

Provided is a medical sheet that increases rigidity of a main body portion constituting the medical sheet and prevents or suppresses twisting when disposed in a biological organ.

The medical sheet includes a sheet-shaped main body portion 10 containing fibers made of a biodegradable material, and the main body portion is formed with a plurality of through-holes 11 and includes a fused portion 50 in which the fibers are gathered and fused around the through-holes.

## Description

### Technical Field

The present invention relates to a medical sheet and a method for producing a medical sheet.

### Background Art

In the medical field, techniques for joining biological organs by surgical surgery (for example, anastomosis of digestive tract) are known. It is known that, when such surgery as described above is performed, it is important as a postoperative prognosis determination factor that no delay in agglutination occurs at a joint portion where biological organs are joined to each other.

Various methods and medical instruments are used in the surgery of anastomosing a biological organ, and for example, a method of suturing a biological organ using a biodegradable suture and a method of using a mechanical anastomosis device for performing anastomosis by a stapler have been proposed. In particular, in the case of performing anastomosis surgery using a mechanical anastomosis device, it is possible to increase a joining force between biological organs at the joint portion as compared with the method using the suture, and thus it is possible to reduce a risk of suture failure.

### Citation List

### Patent Literature

PTL 1: JP2008-516678A

### Summary of Invention

### Technical Problem

In an anastomosis device in Patent Literature 1, in order to prevent leakage, rupture, or the like at an anastomosis site, agglutination of the anastomosis site is promoted by sandwiching a sheet-shaped member (hereinafter referred to as a medical sheet) such as a support structure. Such a medical sheet is usually relatively thin and soft. The present inventors have earnestly studied to increase rigidity of a main body portion constituting a medical sheet, prevent twisting (a state of not being flat due to bending or folding) of the medical sheet when the medical sheet is disposed in a biological organ, and prevent fraying (a state in which fibers are unraveled at a cut end portion of the fibers) of the medical sheet when performing the anastomosis surgery using the mechanical anastomosis device.

Therefore, an object of the invention is to increase rigidity of a main body portion constituting a medical sheet and to prevent or suppress twisting and fraying when the medical sheet is disposed in a biological organ.

### Solution to Problem

The invention is achieved by any one of the following means (1) to (14).
(1) A medical sheet includes a sheet-shaped main body portion containing fibers made of a biodegradable material, and the main body portion is formed with a plurality of through-holes and includes a fused portion in which the fibers are gathered and fused around the through-holes.
(2) In the medical sheet according to (1), the through-hole is formed along a thickness direction of the main body portion, and the fused portion is formed along the thickness direction.
(3) In the medical sheet according to (1) or (2), the fused portion includes a portion where the fibers are completely fused and a portion where peripheries of the fibers are fused while maintaining shapes of the fibers.
(4) In the medical sheet according to (1) or (3), the main body portion includes a vicinity portion located in a vicinity of the through-hole in a plane direction of the main body portion, and a distal portion separated from the through-hole in the plane direction as compared with the vicinity portion, and
   the fibers are present at a higher density in the vicinity portion than in the distal portion.
(5) In the medical sheet according to (4), the fused portion is formed in the vicinity portion.
(6) In the medical sheet according to (4) or (5), the main body portion has a first surface and a second surface formed on a side opposite to the first surface, and
   the fused portion is formed in the vicinity portion and the distal portion on the first surface, and is formed only in the vicinity portion on the second surface.
(7) In the medical sheet according to any one of (1) to (6), the main body portion is formed of a multifilament of the fiber, and at least a part of the multifilament is fused in the fused portion.
(8) A method for producing a medical sheet includes:
   forming a through-hole by a needle penetrating a sheet member containing fibers made of a biodegradable material; and
   forming a fused portion in the fibers around the through-hole by heating the needle in a state in which the needle penetrates the sheet member or by the heated needle penetrating the sheet member.
(9) In the method for producing a medical sheet according to (8), the needle is heated to a temperature equal to or higher than a temperature at which the fibers melt when the needle is heated after the through-hole is formed by the needle penetrating the sheet member or when the heated needle penetrates the sheet member.
(10) In the method for producing a medical sheet according to (8), the needle is heated to a temperature equal to or higher than a melting point of the fibers when the needle is heated after the through-hole is formed by the needle penetrating the sheet member or when the heated needle penetrates the sheet member.
(11) In the method for producing a medical sheet according to any one of (8) to (10), when the needle is heated after the through-hole is formed by the needle penetrating the sheet member, in a state in which a planar base portion on which the needle is provided is in contact with the sheet member, the needle is heated by heating the base portion to form the fused portion.
(12) In the method for producing a medical sheet according to any one of (8) to (10), when the needle is heated after the through-hole is formed by the needle penetrating the sheet member, or when the heated needle penetrates the sheet member, the needle penetrates the sheet member in a state in which a plurality of the sheet members are overlapped, and
   heating of the needle is performed by maintaining the needle at a predetermined temperature for a predetermined time.
(13) In the method for producing a medical sheet according to any one of (8) to (12), the fibers of a plurality of the sheet members are needle-punched before the needle penetrates the sheet member, and the fibers of the plurality of the sheet members are entangled with each other before the needle punching is performed.
(14) In the method for producing a medical sheet according to any one of (8) to (13), the sheet member is cooled after applying heat to the needle, and
   the needle is removed from the sheet member.

### Advantageous Effect of Invention

According to the medical sheet according to (1) to (7) and the method for producing a medical sheet according to (8) to (14), it is possible to increase rigidity of the main body portion constituting the medical sheet, and to prevent or suppress twisting and fraying when disposed in a biological organ.

### Brief Description of Drawings

Fig. 1 is a perspective view illustrating a medical sheet according to an embodiment.
Fig. 2 is an enlarged cross-sectional view illustrating a through-hole of a main body portion of the medical sheet.
Fig. 3 is a diagram illustrating a shape pattern of through-holes of the medical sheet.
Fig. 4 is a diagram illustrating a shape pattern according to a modification of the through-holes of the medical sheet.
Fig. 5 is a diagram illustrating a shape pattern according to a modification of the through-holes of the medical sheet.
Fig. 6 is a diagram illustrating a shape pattern according to a modification of the through-holes of the medical sheet.
Fig. 7 is a perspective view illustrating a medical sheet according to a modification in Fig. 1.
Fig. 8 is an exploded perspective view illustrating a part (distal end portion) of a medical instrument used when a biological organ is anastomosed using the medical sheet.
Fig. 9 is an enlarged image illustrating a fused portion of the medical sheet.
Fig. 10 is an enlarged image illustrating a fused portion of the medical sheet.
Fig. 11 is an enlarged image illustrating a fused portion of the medical sheet.
Fig. 12 is a flowchart illustrating a method for producing the medical sheet according to the embodiment.
Fig. 13 is a view illustrating a needle member used in the method for producing a medical sheet according to a first embodiment.
Fig. 14 is a schematic view illustrating a case where a through-hole is formed in the sheet member by the needle member.
Fig. 15 is a view illustrating a needle member used in the method for producing a medical sheet according to a second embodiment.

### Description of Embodiments

### <First Embodiment>

Hereinafter, embodiments of the invention will be described in detail with reference to the drawings. The embodiments described here are examples for embodying the technical idea of the invention, and do not limit the invention. In addition, other embodiments, examples, operation techniques, and the like that can be conceived by those skilled in the art without departing from the gist of the invention are all included in the scope and the gist of the invention and are included in the inventions described in the claims and their equivalents.

Furthermore, for convenience of illustration and understanding, the drawings attached to the present specification may be changed and schematically expressed from real objects in terms of scale, dimension ratio, shape, and the like as appropriate. These are merely examples, and are not intended to limit the interpretation of the invention.

In the following description, when description is given by adding an ordinal number such as "first" and "second", it is used for convenience and does not define any order unless otherwise specified.

### <Medical Sheet>

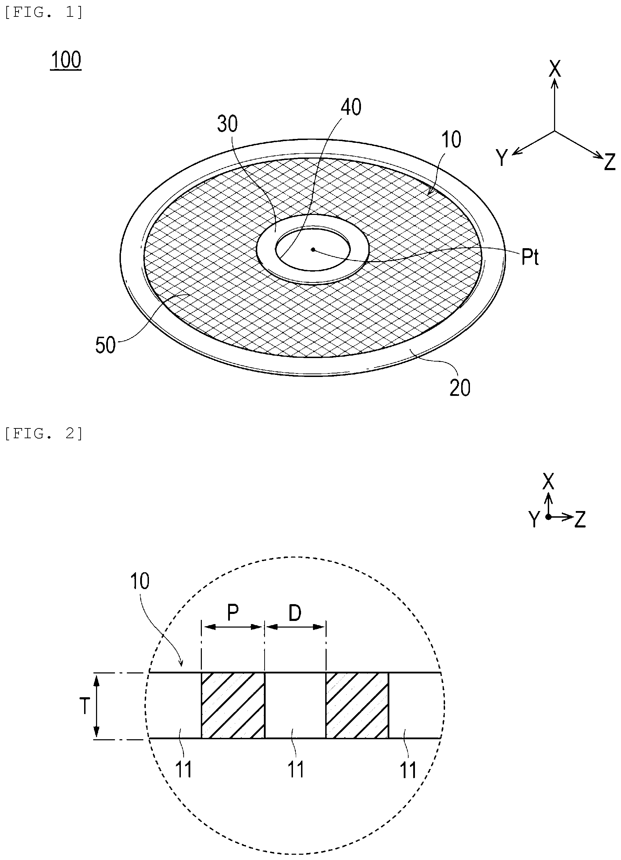

Fig. 1 is a perspective view illustrating a medical sheet 100 according to an embodiment. Fig. 2 is an enlarged view illustrating through-holes 11 of the medical sheet. The medical sheet 100 is sandwiched between two or more biological organs to be anastomosed (one to-be-joined site and the other to-be-joined site), is formed in a flat sheet shape, and has a plurality of through-holes 11. Here, "sandwiched between two or more biological organs to be anastomosed" means at least one of that the medical sheet 100 is disposed in a state of being in direct or indirect contact with the biological organs, that the medical sheet 100 is disposed in a state in which a spatial gap is formed between the medical sheet 100 and the biological organs, or that the medical sheet 100 is disposed in both states (for example, that the medical sheet 100 is disposed in contact with one biological organ and the medical sheet 100 is disposed in a state of not in contact with the other biological organ). Examples of the biological organs include luminal organs such as colon, jejunum, and pancreatic duct. As illustrated in Fig. 1 and the like, the medical sheet 100 includes a main body portion 10, a reinforcing portion 20, a fixing portion 30, a hole 40, and a fused portion 50. The medical sheet 100 may not include the reinforcing portion 20 and the fixing portion 30, and may include the main body portion 10, the hole 40, and the fused portion 50. Alternatively, the medical sheet 100 may not include the reinforcing portion 20, and may include the main body portion 10, the fixing portion 30, the hole 40, and the fused portion 50. In some drawings, an orthogonal coordinate system is illustrated, and in the following, a plane direction of the main body portion 10 is referred to as a plane direction YZ, and a thickness direction is referred to as a thickness direction X. This will be described in detail below.

### <Main Body Portion>

The main body portion 10 is disposed between the biological organs to be anastomosed (for example, colons, pancreatic duct and jejunum), and is formed in a sheet shape capable of following a movement of the biological organs to be anastomosed.

The main body portion 10 is formed in a circular shape as an example as illustrated in Fig. 1, and includes a plurality of through-holes 11 extending in the thickness direction X (axial direction) of the circular shape as illustrated in Fig. 2. For example, a size (hole diameter D) of the through-hole 11 of the main body portion 10 is preferably 0.1 mm to 6 mm, more preferably 0.3 mm to 4 mm, and still more preferably 0.6 mm to 1.5 mm. The main body portion 10 can promote an agglutination effect by the through-holes 11. A ratio of a dimension of the through-hole 11 (distance illustrated in Fig. 2, the hole diameter D of the through-hole 11) to a pitch P (distance illustrated in Fig. 2, a distance between opening edges of two through-holes 11) can be 0.25 or more and less than 40. Since the main body portion 10 includes the plurality of through-holes 11, there are a plurality of values of the hole diameter D corresponding to the respective through-holes 11. Therefore, in the present embodiment, in calculating a value of the ratio described above, an arithmetic mean value of two or more values of the hole diameter D corresponding to the plurality of through-holes 11 is used as a representative value of the hole diameter D. On the other hand, the pitch P of the plurality of through-holes 11 is defined by a shortest distance between openings of the two through-holes 11. However, for a value of the pitch P, there are a plurality of values of the pitch P corresponding to a combination of adjacent through-holes 11. Therefore, in the present embodiment, in calculating the value of the ratio described above, an arithmetic mean value of two or more values of the pitch P corresponding to the combination of the adjacent through-holes 11 is used as a representative value of the pitch P. However, the pitch P described above is an example, and may be periodic or random. The shape of the main body portion 10 as a (perfect) circle is an example, and the shape of the main body portion 10 may instead be an ellipse, a polygon such as a quadrangle, a star, or the like in addition to the above.

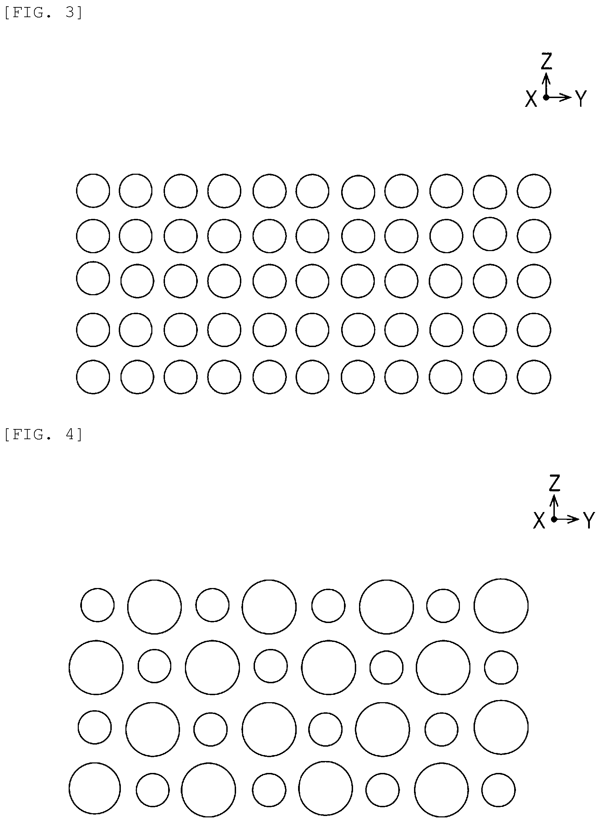

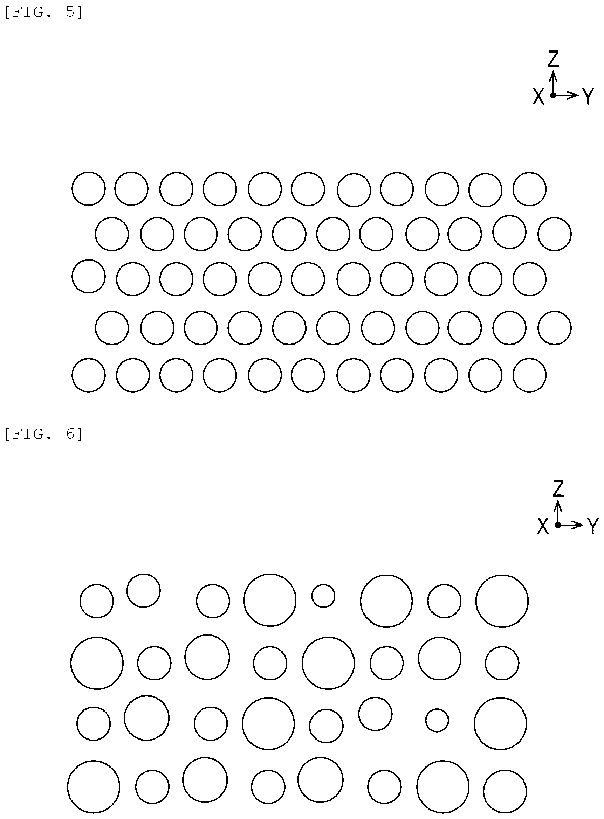

Fig. 3 is a diagram illustrating a shape pattern of the through-holes 11 of the main body portion 10, and Figs. 4 to 6 are diagrams illustrating shape patterns according to modifications of the through-holes 11 of the main body portion 10. In Figs. 2 and 3, the through-holes 11 are illustrated in the same shape and at equal pitches. However, the shape pattern of the through-holes 11 is not limited thereto. In addition to the above, the through-holes 11 having different sizes may be disposed at equal intervals as illustrated in Fig. 4, the through-holes having the same size may be disposed at 60° staggered pitches (staggered arrangement) as illustrated in Fig. 5, or the through-holes 11 having different sizes may be randomly disposed as illustrated in Fig. 6.

A thickness (dimension T illustrated in Fig. 2) of the main body portion 10 is not particularly limited, but is preferably 0.05 mm to 0.7 mm, and more preferably 0.25 mm to 0.45 mm. Numerical values described above for the sizes such as the thickness and the size described above are also examples, and sizes other than those described above may be used. When the dimension T, which is the thickness of the main body portion 10, is 0.7 mm or less (particularly, 0.45 mm or less), flexibility of the main body portion 10 can be increased. Accordingly, the main body portion 10 is in close contact with the biological organs, and followability to the movement of the biological organs is improved. On the other hand, when the dimension T, which is the thickness of the main body portion 10, is 0.05 mm or less, strength of the main body portion 10 is insufficient, and the medical sheet 100 twists and is difficult to be disposed between the biological organs to be anastomosed.

The main body portion 10 can use, as a starting material, a sheet-shaped molded product formed by knitting or weaving a multifilament of a plurality of fibers (for example, strings) formed of a biodegradable material. That is, the main body portion 10 is formed of a biodegradable sheet.
A constituent material from which the main body portion 10 is not particularly limited, and an example thereof includes a biodegradable resin.

Examples of the biodegradable resin include (1) a polymer selected from the group consisting of aliphatic polyester, polyester, polyacid anhydride, polyorthoester, polycarbonate, polyphosphazene, polyphosphate ester, polyvinyl alcohol, polypeptide, polysaccharide, protein, and cellulose; and (2) a copolymer composed of one or more monomers constituting the above (1).

That is, the biodegradable sheet preferably contains at least one biodegradable resin selected from the group consisting of a polymer selected from the group consisting of aliphatic polyester, polyester, polyacid anhydride, polyorthoester, polycarbonate, polyphosphazene, polyphosphate, polyvinyl alcohol, polypeptide, polysaccharide, protein, and cellulose, and a copolymer composed of one or more monomers constituting the polymer. The main body portion 10 is preferably made of a bioabsorbable material such as polyglycolic acid (PGA) or PLGA (polylactic acid-glycolic acid copolymer).

The main body portion 10 is produced by forming a sheet obtained by processing biodegradable fibers into a nonwoven fabric and further forming the through-holes 11 with heating. The main body portion 10 produced in this manner causes a biological reaction by a constituent material such as the biodegradable resin constituting the main body portion 10. The main body portion 10 induces expression of a biological component such as fibrin by this action. The biological component induced in this manner accumulates to penetrate the through-hole 11 of the main body portion 10 from both sides in the plane direction YZ, and thus it is possible to promote agglutination. Therefore, by indwelling the main body portion 10 of the medical sheet 100 in a sandwiched state between biological organs to be joined (for example, between intestinal tracts subjected to anastomosis of a colon, between cut cross sections of the intestinal tracts subjected to anastomosis of the colon, or between a pancreatic parenchyma and a jejunum), the agglutination is promoted by the above mechanism.

### <Reinforcing Portion>

The reinforcing portion 20 is provided to suppress twisting, deviation, breakage, falling off, and the like of the medical sheet 100 when the medical sheet 100 is indwelled between a first to-be-joined site (one to-be-joined site) and a second to-be-joined site (the other to-be-joined site). The reinforcing portion 20 is formed along an outer peripheral edge of a hollow circular shape of the main body portion 10.

In the present embodiment, the reinforcing portion 20 has higher rigidity than the main body portion 10. In the present embodiment, the reinforcing portion 20 has a shape in which the through-holes 11 are not provided in the main body portion 10. The reinforcing portion 20 is preferably made of a bioabsorbable material such as a thermoplastic resin such as polyglycolic acid (PGA), polylactic acid (PLA), polylactic acid-glycolic acid copolymer (PLGA), polydioxanone (PDS), or polycaprolactone (PCL). However, the reinforcing portion 20 may include a non-bioabsorbable material.

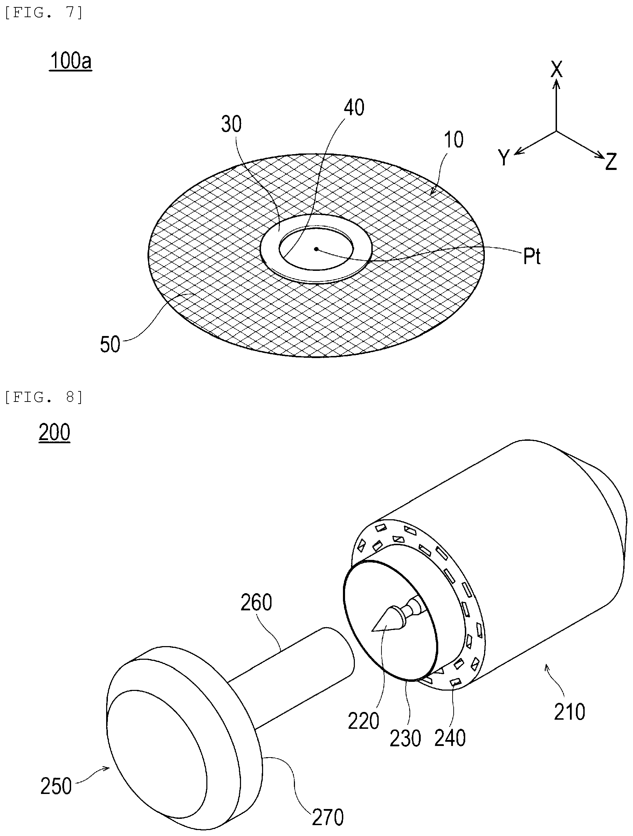

In addition, the reinforcing portion 20 may be provided over an entire periphery on an outward direction side in the plane direction YZ of the main body portion 10, or may be partially provided at one or a plurality of positions in the entire periphery. Here, an outward direction is a direction away from a center portion Pt (virtual point) of the medical sheet 100 in the plane direction YZ. In the plane direction YZ, a direction approaching the center portion Pt of the medical sheet 100 is an inward direction. The reinforcing portion 20 may be joined to the main body portion 10 by an adhesive or heat-welding, or may be sewn on the main body portion 10 by a string or the like. The reinforcing portion 20 may not be provided. Fig. 7 is a perspective view illustrating a medical sheet 100a according to a modification without the reinforcing portion 20. As illustrated in Fig. 7, the medical sheet 100a may include the main body portion 10, the fixing portion 30, the hole 40, and the fused portion 50. When the reinforcing portion 20 is not provided, an outer peripheral edge of the medical sheet 100 is constituted by the main body portion 10 including the through-holes 11 continuous from the main body portion 10 in the outward direction.

Fig. 8 is an exploded perspective view illustrating a distal end portion of a medical instrument 200 used when the medical sheet 100 is disposed in a biological organ. The medical instrument 200 includes a first engagement instrument 210 and a second engagement instrument 250. The medical instrument 200 may also be referred to as a stapler, the first engagement instrument 210 may also be referred to as a trocar, and the second engagement instrument 250 may also be referred to as an anvil. The reinforcing portion 20 and a part of the main body portion 10 on the outward direction side (the main body portion 10 in a vicinity of an inner periphery of the reinforcing portion 20) are integrated with the first to-be-joined site and the second to-be-joined site by staples, which are discharged from a discharge portion 240 of the first engagement instrument 210 of the medical instrument 200, being clamped and deformed together with a clamping portion 270 of the second engagement instrument 250 facing the discharge portion 240.

### <Fixing Portion 30>

The fixing portion 30 is provided to prevent or suppress the deviation of the medical sheet 100 and to prevent the medical sheet 100 from falling off when the medical sheet 100 is indwelled between the first to-be-joined site and the second to-be-joined site. The fixing portion 30 is formed along an inner peripheral edge of the hollow circular shape of the main body portion 10. That is, the fixing portion 30 surrounds the center portion Pt (virtual point) of the medical sheet 100 in the plane direction YZ. Therefore, the medical sheet 100 includes the circular reinforcing portion 20 surrounding the center portion Pt of the medical sheet 100, the main body portion 10 surrounding an outer periphery of the reinforcing portion 20, and the reinforcing portion 20 surrounding an outer periphery of the main body portion 10. In the medical sheet 100, the fixing portion 30, the main body portion 10, and the reinforcing portion 20 are disposed in this order in the outward direction of the plane direction YZ from the center portion Pt.

Similarly to the reinforcing portion 20, the fixing portion 30 has a shape in which the through-holes 11 are not provided in the main body portion 10. The fixing portion 30 can be formed of the same material as the reinforcing portion 20.

The fixing portion 30 may be provided over an entire periphery of the main body portion 10 on an inward direction side in the plane direction YZ, or may be partially provided at one or a plurality of positions in the entire periphery. Further, the fixing portion 30 is formed coaxially with an inner edge portion, but a center position may be shifted from the main body portion 10 if the fixing portion 30 does not enter an agglutination region. The fixing portion 30 and the inward direction side of the main body portion 10 are separated from the outward direction side of the main body portion 10 and the reinforcing portion 20 by being punched out by a punching portion 230 of the first engagement instrument 210 of the medical instrument 200 during surgery. The fixing portion 30 may not be provided. When the fixing portion 30 is not provided, an inner peripheral edge of the medical sheet 100 is constituted by the main body portion 10 including the through-holes 11 continuous from the main body portion 10 in the inward direction.

### <Hole>

The hole 40 is separated from an outer peripheral edge portion of the main body portion 10 in the plane direction YZ of the main body portion 10, and is defined by the fixing portion 30 in the present embodiment. The hole 40 allows insertion of a shaft 260 of the second engagement instrument 250 of the medical instrument 200. In the present embodiment, since the hole 40 has a hole diameter larger than the hole diameter D of each through-hole 11, the hole 40 allows the insertion of the shaft 260. The shaft 260 of the second engagement instrument 250 can accommodate a positioning portion 220 of the first engagement instrument 210.

The hole 40 is formed in a substantially circular shape when viewed from the thickness direction X in the present embodiment. However, a specific shape of the hole is not limited to the circular shape as long as the main body portion 10 can promote the agglutination of biological tissues. A cross section of the hole 40 is preferably a perfect circle, but may instead be a notch having a linear shape, an elliptical shape, a triangular shape, a quadrangular shape, a concave shape, a convex shape, a cross shape, or the like other than the perfect circle.

### <Fused Portion>

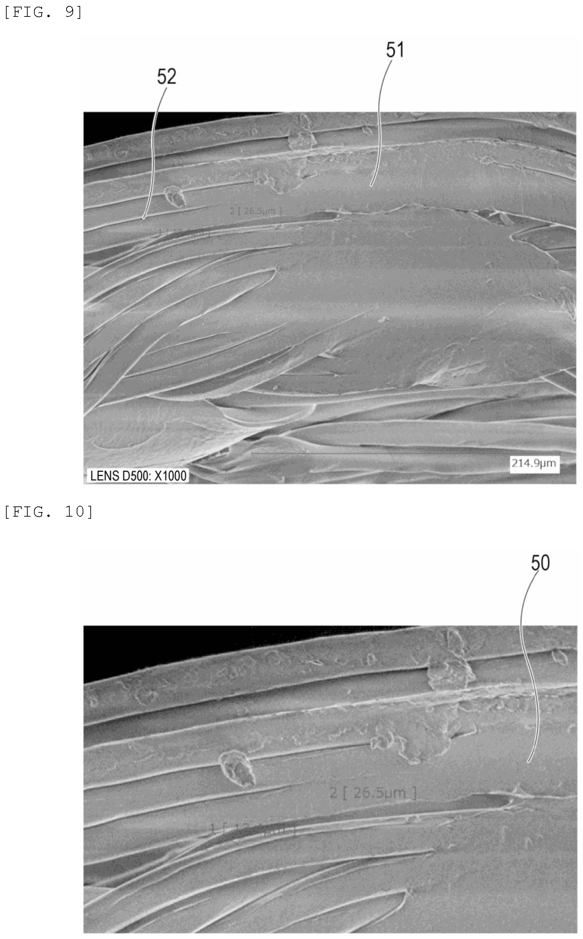

Figs. 9 to 11 are images illustrating a part of the fused portion 50. The fused portion 50 is formed as a site where fibers are gathered and fused around the through-hole 11. The through-hole 11 of the main body portion 10 is formed along the thickness direction X of the main body portion 10. The fused portion 50 is formed along the thickness direction X.

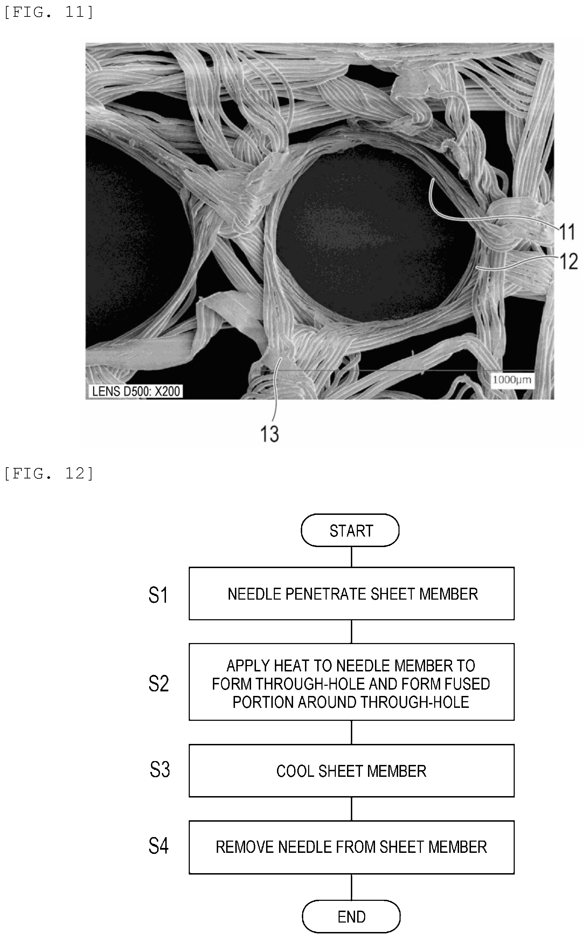

As illustrated in Fig. 9, the fused portion 50 as illustrated in Fig. 10 includes a first portion 51 in which the fibers are completely fused, and a second portion 52 in which peripheries of the fibers are fused while maintaining shapes of the fibers. Here, "completely fused" means a state in which the fibers are completely integrated (integrated to such an extent that the fibers, which are originally separate, cannot be distinguished even by checking with a microscope or the like).
"Peripheries of the fibers are fused while maintaining shapes of the fibers" means a state in which the fibers are not completely integrated (integrated to such an extent that the fibers, which are originally separate, cannot be distinguished even by checking with a microscope or the like), but a portion is included in which the peripheries of the fibers are melted and the fibers are integrated (integrated to such an extent that the fibers, which are originally separate, can be distinguished even by checking with a microscope or the like). As illustrated in Fig. 11, the main body portion 10 includes a vicinity portion 12 located in the vicinity of the through-hole 11 in relation to the fused portion 50, and a distal portion 13 separated from the through-hole 11 in the plane direction YZ as compared with the vicinity portion 12. The vicinity portion 12 includes an opening edge of the through-hole 11. The distal portion 13 includes a center vicinity of the distance (pitch P) between two through-holes 11. Here, in the main body portion 10, a range from the edge portion of one through-hole 11 to less than 50% of a length between adjacent through-holes 11 can be the vicinity portion 12, and the other range can be the distal portion 13. The vicinity portion 12 is configured such that the fibers are present at a higher density than the distal portion 13.

As will be described later, the fused portion 50 is formed in the vicinity portion 12 and the distal portion 13 on one side (first surface) in the thickness direction X, and is formed only in the vicinity portion 12 on the other side (second surface opposite to the first surface). The fused portion 50 can be formed such that at least a part of multifilaments in which a plurality of fibers are collected are fused. However, the fused portion 50 may be formed such that at least a part of the fibers (strings) of the multifilament are fused. A size of the fused portion 50 is not particularly limited, and can be 0.015 mm to 0.7 mm, for example. The fused portion 50 can have a ratio of 3% or more and 100% or less to a non-melted site. In addition, an occupancy rate of the fused portion 50 with respect to the main body portion 10 when the main body portion 10 is viewed in a plan view (viewed from the thickness direction X) can be 0.002% or more.

### <Method for Forming Fused Portion>

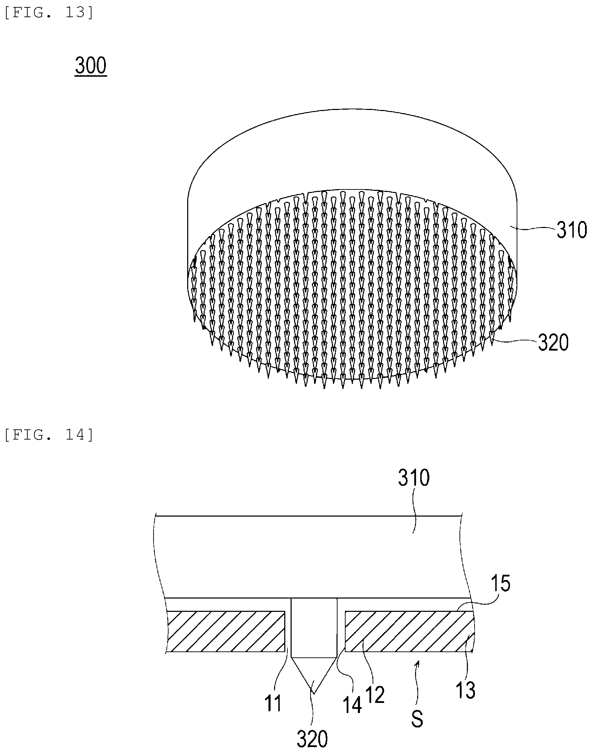

Next, a method for forming the fused portion 50 in the medical sheet 100 will be described. Fig. 12 is a flowchart illustrating a method for forming the medical sheet 100 according to the embodiment. Fig. 13 is a diagram illustrating a needle member 320 used in the method for forming the fused portion 50 according to the first embodiment. Fig. 14 is a diagram illustrating a case where the through-hole 11 is formed in a sheet member S used when the fused portion 50 is formed in the medical sheet 100. The sheet member S is a sheet-shaped member including fibers made of a biodegradable material, in which the through-holes 11 are not formed.

As an example of the method for forming the fused portion 50, as illustrated in Fig. 13, a forming member 300, in which an large number of needle members 320 having thermal conductivity are provided at a tip of a base portion 310 having a circular plane serving as a base, is used. A member that can be heated by generating ultrasounds or the like is incorporated in the base portion 310 of the forming member 300, and heat can be conducted to the plurality of needle members 320 by generating heat in the base portion 310. In the present embodiment, the forming member 300 is heated in advance. The needle members 320 are heated to a temperature equal to or higher than a temperature at which fibers made of a biodegradable material forming the main body portion 10 melt, or equal to or higher than a melting point of the fibers (about 200°C, about 218°C, or 218°C or higher in the case of PGA, or about 200°C, about 215°C, or 215°C or higher in the case of PLGA). Then, the needle members 320 are inserted into the sheet member S before processing of the medical sheet 100 (S1), and the base portion 310 is brought into contact with the sheet member S to perform pressing.

Accordingly, as illustrated in Fig. 14, a periphery of the edge portion (corresponding to the vicinity portion 12 of the main body portion 10) of the through-hole 11 formed in the sheet member S by being perforated by the needle member 320 is heated and melted by the needle member 320 to which heat is applied, and the fibers around the through-hole 11 are melted to be integrated, thereby forming the fused portion 50 (S2). The needle member 320 in the present embodiment has a shape in which a columnar portion and a conical portion having the same diameter are overlapped on the base portion 310, but the shape of the needle member 320 is not particularly limited as long as the through-hole 11 can be formed with the target hole diameter D. The needle member 320 may have a conical shape, a pyramid shape, a cylindrical shape, a tapered shape, or the like. The needle member 320 may have a diameter equal to or equivalent to the hole diameter D. In the present embodiment, the diameter of the columnar portion of the needle member 320 and the diameter of a bottom surface of the conical portion have the same diameter as the hole diameter D, and the columnar portion penetrates and perforates the sheet member S. However, as long as the through-hole 11 can be formed with the target hole diameter D, the columnar portion may not penetrate the sheet member S.

A site 14 in Fig. 14 indicates a site of the sheet member S in direct contact with the needle member 320, and a site 15 indicates a region (first surface) in direct contact with the base portion 310. The sites 14 and 15 in direct contact with the base portion 310 and the needle member 320 has a larger thermal load than a site (second surface) not in direct contact with the base portion 310 and the needle member 320. Therefore, when the sheet member S is viewed from the thickness direction X, the fibers are melted more on one side (front side, the sites 14 and 15 in direct contact with the base portion 310 and the needle member 320, the first surface) than on the other side (back side, the site not in direct contact with the base portion 310 and the needle member 320, the second surface), and the rigidity is increased.

In addition, as illustrated in Fig. 14, since a surface (site 15) of the sheet member S in contact with the base portion 310 is a non-uniform plane due to the fibers, there is a portion not in direct contact with the base portion 310. The through-hole 11 is formed while avoiding fibers of the sheet member S at an edge portion (corresponding to the vicinity portion 12 of the main body portion 10) perforated by the needle member 320. Accordingly, a fiber density of the vicinity portion 12 of the through-hole 11 is higher than that of the distal portion 13. That is, a density at the through-holes 11 is higher than a density at the surface of the sheet member S. Since the site 15 where the fibers are partially melted and fused by being heated by contact with the base portion 310 is formed only on one surface of the main body portion 10, the main body portion 10 can have both flexibility and rigidity to a certain extent. The fused portion 50 is formed in the vicinity portion 12 and the distal portion 13 on one side (the front side, the sites 14 and 15 in direct contact with the base portion 310 and the needle member 320, the first surface) when viewed from the thickness direction X where the base portion 310 is in direct contact, and the fused portion 50 is formed only in the vicinity portion 12 on the other side (the back side, the site not in direct contact with the base portion 310 and the needle member 320, the second surface) where the base portion 310 and the needle member 320 are not in direct contact. Although a site (corresponding to the distal portion 13 of the main body portion 10) separated from the vicinity of the needle member 320 is not completely melted by heat, the portion is slightly melted by heat conduction, and the fused portion 50 is formed in a state in which the fibers are not completely melted. The fused portion 50 may be formed by fusing strings between multifilaments, or may be formed by fusing bundled multifilaments. However, it can be expected that the fusion of the bundled multifilaments increases the rigidity.

The number of the sheet members S used for forming the main body portion 10 is not particularly limited, and may be one or two or more, but is preferably 2 to 8, and more preferably 4 since a balance between a heating condition and the strength is good. By forming the through-hole 11 while applying heat, the periphery of the edge portion is melted, and the shape of the through-hole 11 can be easily maintained.

After applying heat to the needle member 320, the sheet member S is cooled (S3), and the needle member 320 is removed from the sheet member S (S4). A cooling method is not particularly limited, and natural cooling or forced cooling by air or the like may be performed. Further, cooling may be performed while the needle member 320 is inserted or may be performed after the needle member 320 is removed from the sheet member S. When the heated needle member 320 is punctured and processed as described above and the needle member 320 is separated from the sheet member S and cooled, a protrusion may be formed around the edge portion. This protrusion facilitates positioning. Further, hardness of the sheet member S and the sizes and distribution of the through-holes can be adjusted by changing a temperature, time, and pressure for hot pressing, and the diameter and pitch of the needle members.

As described above, the medical sheet 100 according to the present embodiment includes the sheet-shaped main body portion 10 containing the fibers made of the biodegradable material. The main body portion 10 includes the fused portion 50 in which the plurality of through-holes 11 are formed and fibers are gathered and fused around the through-holes 11. With such a configuration, the shape of the through-hole 11 of the main body portion 10 can be easily maintained. In addition, by having the configuration of the fused portion 50 as described above, the rigidity of the main body portion 10 can be increased, and an occurrence of twisting or deviation of the medical sheet 100 can be prevented or suppressed. In addition, by increasing the rigidity of the main body portion 10, it is possible to prevent or suppress fraying when the medical sheet 100 is punched by the stapler such as the medical instrument 200, and to facilitate punching.

The through-holes 11 are formed along the thickness direction X of the main body portion 10. The fused portion 50 is formed along the thickness direction X. Therefore, the rigidity of the main body portion 10 can be easily increased to contribute to preventing or suppressing the occurrence of twisting of the medical sheet 100.

The fused portion 50 includes the first portion 51 in which the fibers are completely fused, and the second portion 52 in which the peripheries of the fibers are fused while maintaining the shapes of the fibers. As described above, since not only the second portion 52 but also the first portion 51 is present in the fused portion 50, the biological component easily enters a gap between the fibers, and the agglutination effect can be easily exhibited.

The main body portion 10 includes the vicinity portion 12 located in the vicinity of the through-hole 11 in the plane direction YZ of the main body portion 10, and the distal portion 13 separated from the through-hole 11 in the plane direction YZ as compared with the vicinity portion 12. The vicinity portion 12 has such a configuration that the fibers are present at a higher fiber density than the distal portion 13 by perforating the needle member 320 by the above method and positioning the fibers to avoid the surroundings. With such a configuration, the strength of the main body portion 10 can be easily improved, and thus the shape of the through-hole 11 can be easily maintained.

In addition, the fused portion 50 is formed in the vicinity portion 12 and the distal portion 13 at the site 15 (first surface) which is a side directly in contact with the base portion 310 and the needle member 320 in the thickness direction X of the main body portion 10. The fused portion 50 is formed only in the vicinity portion 12 on the side (second surface) opposite to the site 15 in the thickness direction X. With such a configuration, the shape of the through-hole 11 can be easily maintained, and fraying when the medical sheet 100 is punched by the medical instrument 200 can be prevented or suppressed.

The main body portion 10 is made of a multifilament of fibers. The fused portion 50 can be formed such that at least a part of the multifilaments is fused. With such a configuration, the strength of the main body portion 10 can be easily improved.

In addition, in a method for producing the medical sheet 100, the heated needle member 320 penetrates the sheet member S containing the fibers formed of the biodegradable material to form the through-hole 11, and the heated needle member 320 forms the fused portion 50 in the fibers around the through-hole 11. With such a configuration, it is possible to produce the medical sheet 100 in which the rigidity of the main body portion 10 is high and twisting hardly occurs.

When the heated needle member 320 penetrates the sheet member S, the needle member 320 is heated to a temperature equal to or higher than the melting point of the fibers or a temperature at which the fiber melts. With such a configuration, the periphery of the through-hole 11 can be melted to form the fused portion 50 in the fibers around the through-hole 11.

In the present embodiment, in a state in which the base portion 310 on which the needle member 320 is provided is in contact with the sheet member S, the base portion 310 is heated in advance, so that the needle member 320 is heated to form the fused portion 50. With such a configuration, it is possible to form the fused portion 50 that can increase the rigidity of the main body portion 10 and make it difficult for twisting to occur.

In the present embodiment, the sheet member S is cooled after heat is applied to the needle member 320, and the sheet member S is removed from the needle member 320. With such a configuration, the medical sheet 100 in which the fused portion 50 is formed can be obtained.

### (Second Embodiment)

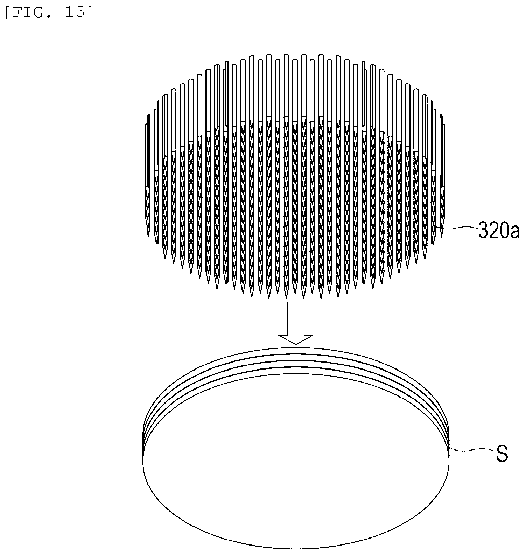

Fig. 15 is a schematic diagram illustrating a producing method according to the second embodiment for forming the fused portion 50 of the medical sheet 100. In the present embodiment, the method for producing the medical sheet 100 is different from that of the first embodiment, but the medical sheet 100 itself is the same as that of the first embodiment, and so a detailed description of the medical sheet 100 is not repeated.

In the present embodiment, as illustrated in Fig. 15, a plurality of sheet members S containing the biodegradable material are overlapped, and a plurality of needle members 320a having thermal conductivity without the base portion 310 puncture the sheet members to form the through-holes 11 in the sheet members S with respect to the forming member 300 described in the first embodiment (S1). Afterwards, the resulting plurality of overlapped sheet members S containing the biodegradable material may have different shape patterns of the through-holes 11. For example, a plurality of sheet members S having the through-holes 11 disposed in two or more different shape patterns may be selected from the shape patterns of the through-holes 11 of the main body portion 10 illustrated in Figs. 3 to 6 and overlapped.

Then, the needle members 320a are maintained at a high temperature for a predetermined time in a state in which the needle members 320a puncture the sheet members S. In the present embodiment, the predetermined temperature can be set to the above-described temperature at which the fibers melt or any above-described temperature equal to or higher than the melting point of the fibers. Accordingly, the fused portion 50 is formed in the sheet members S (S2). In the present embodiment, the fused portion 50 is formed (only) in the vicinity portion 12 on any side (first surface and second surface) in the thickness direction X. After heating the needle members 320a, the sheet members S are cooled (S3), and the needle members 320a are removed from the sheet members S (S4).

As described above, in the present embodiment, the fused portion 50 is formed in the vicinity portion 12 on any side in the thickness direction X. With such a configuration, the shape of the through-hole 11 can be easily maintained.

When the needle members 320a are heated after the needle members 320a penetrate the sheet members S to form the through-hole 11, the needle members 320a are maintained at the temperature at which the fibers melt or at the temperature equal to or higher than the melting point. With such a configuration, the periphery of the through-hole 11 can be melted to form the fused portion 50 in the fibers around the through-hole 11.

In addition, when the needle members 320a penetrate the sheet members S, the needle members 320a penetrate the sheet members S in a state in which a plurality of sheet members S are overlapped. Then, in the state in which the needle members 320a penetrate the sheet members S, a state in which the needle members 320a are heated to a high temperature is maintained for a predetermined time. With such a configuration, it is also possible to obtain the medical sheet 100 in which the fused portion 50 is formed in the fibers constituting the sheet members S.

The invention is not limited to the above-described embodiments, and various modifications can be made within the scope of the claims. The first embodiment describes that the needle member 320 heated in advance punctures the sheet member S, but the fibers may be processed by needle punching to be entangled with each other more than before the needle punching is performed. Accordingly, entanglement between the fibers is promoted, and it can be expected that the rigidity of the main body portion 10 is further increased when the fibers are fused. Further, needle punching may be performed on the sheet member S before the fused portion 50 is formed in the second embodiment. By performing needle punching, the entanglement between fibers of the plurality of sheet members S can be promoted. In addition, by performing needle punching, a multifilament in which a plurality of fibers are collected can be loosened, and the entanglement between the fibers can be promoted. By promoting the entanglement of the fibers, the fused portion 50 is easily formed when the fibers are fused, and the rigidity of the medical sheet 100 can be increased.

In addition, the first embodiment describes that the needle member 320 heated in advance punctures the sheet member S to form the fused portion 50, but the base portion 310 may be heated after the unheated needle member 320 punctures the sheet member S. With such a configuration, it is also possible to form the fused portion 50 that can increase the rigidity of the main body portion 10 and make it difficult for twisting to occur. In the second embodiment, the needle members 320a are maintained at the high temperature for the predetermined time after the needle members 320a puncture the sheet members S, but the needle members 320a may be heated before the needle members 320a puncture the sheet members S.

The present application is based on Japanese patent application No. 2023-164236 filed on September 27, 2023, the entire content of which is incorporated herein by reference.

### Reference Signs List

10: main body portion
11: through-hole
12: vicinity portion
13: distal portion
50: fused portion
51: first portion
52: second portion
100, 100a: medical sheet
310: base portion
320, 320a: needle member
S: sheet member
X: thickness direction
YZ: plane direction

## Claims

1. A medical sheet comprising:
a sheet-shaped main body portion containing fibers made of a biodegradable material, wherein
the main body portion is formed with a plurality of through-holes and includes a fused portion in which the fibers are gathered and fused around the through-holes.

2. The medical sheet according to claim 1, wherein
the through-holes extend along a thickness direction of the main body portion, and
the fused portion extends along the thickness direction.

3. The medical sheet according to claim 1, wherein
the fused portion includes a portion where the fibers are completely fused and a portion where peripheries of the fibers are fused while maintaining shapes of the fibers.

4. The medical sheet according to claim 1, wherein
the main body portion includes a vicinity portion located in a vicinity of the through-hole in a plane direction of the main body portion, and a distal portion separated from the through-hole in the plane direction as compared with the vicinity portion, and
the fibers are present at a higher density in the vicinity portion than in the distal portion.

5. The medical sheet according to claim 4, wherein
the fused portion is formed in the vicinity portion.

6. The medical sheet according to claim 4, wherein
the main body portion has a first surface and also has a second surface on a side off the main body portion opposite to the first surface, and
the fused portion is in the vicinity portion and in the distal portion on the first surface of the main body portion, and is only in the vicinity portion on the second surface of the main body portion.

7. The medical sheet according to claim 1, wherein
the main body portion is comprised of a multifilament of the fibers, and at least a part of the multifilament is fused in the fused portion.

8. A method for producing a medical sheet, the method comprising:
forming a through-hole by a needle penetrating a sheet member containing fibers made of a biodegradable material; and
forming a fused portion in the fibers around the through-hole by heating the needle in a state in which the needle penetrates the sheet member or by the heated needle penetrating the sheet member.

9. The method for producing a medical sheet according to claim 8, wherein
the needle is heated to a temperature equal to or higher than a temperature at which the fibers melt when the needle is heated after the through-hole is formed by the needle penetrating the sheet member or when the heated needle penetrates the sheet member.

10. The method for producing a medical sheet according to claim 8, wherein
the needle is heated to a temperature equal to or higher than a melting point of the fibers when the needle is heated after the through-hole is formed by the needle penetrating the sheet member or when the heated needle penetrates the sheet member.

11. The method for producing a medical sheet according to claim 8, wherein
when the needle is heated after the through-hole is formed by the needle penetrating the sheet member, in a state in which a planar base portion on which the needle is provided is in contact with the sheet member, the needle is heated by heating the base portion to form the fused portion.

12. The method for producing a medical sheet according to claim 8, wherein
when the needle is heated after the through-hole is formed by the needle penetrating the sheet member, or when the heated needle penetrates the sheet member, the needle penetrates the sheet member in a state in which a plurality of the sheet members are overlapped, and
heating of the needle is performed by maintaining the needle at a predetermined temperature for a predetermined time.

13. The method for producing a medical sheet according to claim 8, wherein
the fibers of a plurality of the sheet members are needle-punched before the needle penetrates the sheet members, and the fibers of the plurality of the sheet members are entangled with each other before the needle punching is performed.

14. The method for producing a medical sheet according to claim 8, wherein
the sheet member is cooled after applying heat to the needle, and
the needle is removed from the sheet member.
